# EUROPEAN PATENT APPLICATION

(11) **EP 4 501 338 A1**
(43) Date of publication of application: **05.02.2025**
(21) Application number: 23780281.4
(22) Date of filing: 26.03.2023
(51) Int. Cl.: A61K 35/32, A61K 9/08, A61K 31/7105, A61K 48/00, A61P 19/02, A61P 35/00, C12N 5/077, C12N 5/10, C12N 15/113, C12N 15/88

(54) **PHARMACEUTICAL COMPOSITION CONTAINING MIR-140 AND METHOD FOR PRODUCING SAME**

(30) Priority: 28.03.2022 JP 2022051095
(71) Applicant: Hiroko Science Inc., Fukuoka-shi, Fukuoka 810-0001 (JP)
(72) Inventor: YANAGA Hiroko, Fukuoka-shi, Fukuoka 810-0001 (JP); NISHINA Hiromichi, Fukuoka-shi, Fukuoka 812-0069 (JP); YANAGA Katsu, Fukuoka-shi, Fukuoka 810-0001 (JP)
(74) Representative: van Dam, Vincent
(86) International application number: PCT/JP2023/012062
(87) International publication number: WO 2023/190271

(57) **Abstract**

[Problem] To provide: a pharmaceutical composition containing, as an active ingredient, exosomes containing a large amount of miR-140; and a method for producing the same. [Solution] A pharmaceutical composition containing exosomes derived from auricular chondrocytes, the exosomes containing miR-140; and a method for producing a pharmaceutical composition, the method including an exosome acquisition step for obtaining exosomes from auricular chondrocytes. The exosome acquisition step includes an auricular chondrocyte culture step for culturing auricular chondrocytes to obtain cultured chondrocytes, and an exosome collection step for collecting exosomes from the cultured chondrocytes cultured at the auricular chondrocyte culture step. At the auricular chondrocyte culture step, the medium in which the auricular chondrocytes are cultured contains estrogen.

## Description

### Technical Field

The invention relates to a pharmaceutical composition containing miR-140 and a method for producing the same.

### Background Art

Japanese Patent No. 6726726 describes a pharmaceutical composition having exosomes containing miR-140-3p (claim 2). The exosomes in this publication are collected from cardiosphere-derived cells (CDCs). Japanese Patent Nos. 6035010 and 5395439 also describe pharmaceutical compositions containing miR-140.

### Citation List

### Patent Literature

Patent Literature 1: Japanese Patent No. 6726726.
Patent Literature 2: Japanese Patent No. 6035010.
Patent Literature 3: Japanese Patent No. 5395439.

### Summary of Invention

### Technical Problem

The invention aims to provide a pharmaceutical composition containing exosomes that contain miR-140 as an effective ingredient and a method for producing a pharmaceutical composition containing exosomes that contain a large amount of miR-140.

### Solution to Problem

The first invention relates to a pharmaceutical composition containing exosomes derived from auricular chondrocytes. The exosomes contain miR-140.

The next invention relates to a method for producing the above-mentioned pharmaceutical composition.

The method comprises an exosomes obtaining step of obtaining exosomes from auricular chondrocytes.

### Advantageous Effects of Invention

The invention can provide a pharmaceutical composition containing exosomes that contain a large amount of miR-140 as an effective ingredient and a method for producing the same.

### Description of Embodiments

Embodiments of the present invention will be explained below. The present invention is not limited to the embodiments described below and incorporates appropriate modifications from the embodiments below to the extent that they are obvious to those skilled in the art.

The first invention relates to a pharmaceutical composition containing exosomes derived from auricular chondrocytes. The exosomes contain miR-140.

### Auricular chondrocytes

The method of collecting auricular chondrocytes is known, for example, as described in Japanese Patent No. 5320526. Auricular chondrocytes of female origin are preferable as auricular chondrocytes.

### Pharmaceutical composition that contains exosomes

Exosomes are also called exosome. Exosomes are lipid bilayer particles that do not have a nucleus and are released from a cell. Exosomes encapsulate a nucleic acid material such as mRNA or miRNA. For example, Japanese Patent No. 6524162 describes a pharmaceutical composition containing mesenchymal stem cell exosomes as an effective ingredient. As described above, exosomes are used as an effective ingredient in compositions for the treatment or prevention of various diseases.

### miR-140

miRNA is a type of small RNA of 18-22 bases that binds to mRNA in a cell and regulates its transcription. miR-140 (such as miR-140-3p and miR-140-5p) is a known miR. For example, miR-140 is described in Japanese Patent Nos. 6948059, 6726726 and 6800470. Pharmaceutical compositions containing miRNA are also known, for example, as described in Japanese Patent No. 6651061.

### Target diseases of the pharmaceutical composition

Examples of target diseases of the pharmaceutical composition are solid tumors and cancers, as described in the above patent literature. Examples are brain tumor, pharyngeal cancer, maxillary cancer, oral cancer, tongue cancer, lip cancer, thyroid cancer, lung cancer, breast cancer, esophageal cancer, stomach cancer, large intestine cancer, hepatocellular cancer, biliary tract cancer, pancreatic cancer, renal cell cancer, bladder cancer, cervical cancer, uterine corpus cancer, ovarian cancer, skin cancer, malignant melanoma, myeloid tumor, osteosarcoma, soft tissue tumor, angiosarcoma and pediatric solid tumor. The pharmaceutical composition is also considered to be effective in the treatment of osteoarthritis.

### Ingredients of the pharmaceutical composition

The pharmaceutical composition may contain known carriers (for example, water, normal saline, cream or excipients). The pharmaceutical composition may also contain known materials as appropriate. For example, the pharmaceutical composition may also contain known anticancer drugs. The pharmaceutical composition can be obtained by mixing effective ingredients with a carrier as appropriate.

A route of administration of the pharmaceutical composition of the present invention is not limited and may be systemic or local administration.

A dosage form of the pharmaceutical composition of the present invention may be determined as appropriate according to the route of administration and includes but is not limited to injectable, intravenous, tablet, capsule, fine granule, powder, liquid, solution dissolved in syrup and the like, patch, suppository, etc.

Subjects to which the pharmaceutical composition of the present invention is administered are not limited, and for example, the present invention can be administered to mammals (humans, pigs, cows, monkeys, baboons, dogs, cats, rats, mice, etc.).

Methods of administration (route of administration, dosage, number of administration times per day, timing of administration, etc.) of the pharmaceutical composition of the present invention to a subject are not limited and can be determined as appropriate by a person skilled in the art (for example, a physician) according to the subject's health condition, severity of disease, type of concomitant drug, etc. An example of a dosage of miR-140 is 1 µg or more and 1 mg or less/day.

The next invention relates to a method for producing the above-mentioned pharmaceutical composition. The method comprises an exosomes obtaining step of obtaining exosomes from auricular chondrocytes.

A preferable example of the exosomes obtaining step includes a culturing step of auricular chondrocytes and an exosomes collecting step.

The culturing step of the auricular chondrocytes is a step of culturing auricular chondrocytes to obtain cultured chondrocytes. A culture medium for culturing auricular chondrocytes preferably contains estrogen. The estrogen preferably contains estradiol. The auricular chondrocytes can be isolated, for example, by removing skin, connective tissue and cartilage membrane from an auricle of a human, animal or the like, cutting it into small pieces with a size of about 5 mm x 5 mm, and then treating it with collagenase. Isolated auricular chondrocytes may be used as is or used for the pharmaceutical composition after culturing as appropriate.

### Culturing

Auricular chondrocytes can be cultured under known culture conditions using a known culture medium. Note that estrogen (especially estradiol) is preferably added to a culture system.

The exosomes collecting step is a step of collecting exosomes from the cultured chondrocytes cultured in the culturing step of the auricular chondrocytes. The exosomes collecting step preferably includes a step of collecting exosomes from cultured chondrocytes using monensin or a monensin salt.

### Collecting exosomes

The exosomes can be collected according to the method described in, for example, Japanese Patent No. 6524162.

### Monensin

Monensin is a polyether antibiotic isolated from a genus Streptomyces by bacteria. This molecule is very widely used as an additive in ruminant feed (Rumensin, a registered trademark) to prevent coccidial infections. Examples of monensin salts are sodium and potassium salts.

Another preferable example of the exosomes obtaining step other than the above includes a culturing step of culturing auricular chondrocytes, an immortalization step, and an exosomes collecting step.

The immortalization step is a step of immortalizing cultured chondrocytes cultured in the culturing step of the auricular chondrocytes to obtain immortalized cells.

### Immortalization step

Lentiviral infection can be induced to immortalize cultured chondrocytes. For example, the step of immortalization preferably comprises a step of immortalizing cultured chondrocytes using SV40 or hTERT. Immortalization using a lentivirus is described, for example, in Japanese Patent Nos. 6302356 and 6868565. Cultured chondrocytes can be immortalized by referring to the above patent literature and examples described in the above literature.

In this example, the exosomes collecting step is a step of collecting exosomes from immortalized cells. The exosomes collecting step preferably includes a step of collecting exosomes from immortalized cells using monensin or a monensin salt.

This specification also provides methods of treating and preventing target diseases comprising a step of administering the above-mentioned pharmaceutical composition to a human (for example, a patient).

### Example 1

### Immortalization of human auricular chondrocytes

### Lentiviral infection

Normal female auricular chondrocytes were seeded in a 6-well plate at a confluency of 30-40% 24 hours prior to infection.

The cells were infected the next day with Lenti-SV40 (LV613) and Lenti-hTERT (LV615) lentiviruses at an MOI of 5.

After 72 hours, cells were passaged and checked for gene transfer by qRT-PCR.

RNAs were extracted from infected and uninfected cells using 5X All-In-One Mastermix and EXCellentCT Lysis Kit (G916) to synthesize cDNAs.

Gene transfer was detected in the above-mentioned cDNAs by qRT-PCR using
SV40 Forward Primer:
   5'GACTCAGGGCATGAAACAGG3' (sequence No. 1) and
SV40 Reverse Primer:
   5' ACTGAGGGGCCTGAAATGA 3' (sequence No. 2).

The results showed that the gene was not transferred to uninfected cells but was transferred to infected cells. The cell morphology of the infected cells did not differ much from that of the uninfected cells. However, when the cells continued to be passaged, the uninfected cells stopped growing at 15 passages, while the infected cells remained alive past 25 passages. Therefore, it was concluded that the infection was successful.

### Example 2

Addition of various exosome release enhancers to immortalized chondrocytes and treatment of culture supernatant

The fifth passage of immortalized chondrocytes was passaged and seeded in four flasks with a volume of 75 cm² at a concentration of 1.5 x 10⁶ cells per flask. Culturing was started after adding 10 nM of estradiol to three of them and without adding it to one of them. On day four of culturing, 10 uM of monensin, 20 nM of aplimod and 10 uM of D609 were added to each of the flasks with estradiol as exosome release enhancers, and the flasks were kept cultured until the next day using the flask without estradiol as a control. The medium was collected from each flask the following day and then centrifuged at 1,500 rpm for 5 min. The supernatants were further filtered through a filter with a pore size of 0.22 um and stored as exosomal fractions at -80°C until an analysis of miR.

### Example 3

### Extraction of miRs from samples and identification of extracted nucleic acids

MicroRNAs were extracted from the exosomal fractions in Example 2 using an exoRNAeasy Serum/Plasma Maxi Kit (Qiagen). The A260/A280 and A260/A230 ratios of the extracted RNAs were measured using a spectrophotometer Nano Drop One (Thermo Fisher Scientific), showing that the samples were free of protein and salt impurities. Electrophoresis of the samples was further conducted using Agilent 2100 Bioanalyzer (Aligent Technologies), confirming that only the target nucleic acids were contained. As shown in the table below, the amount of nucleic acid collected was found to be the highest in the case of treatment with monensin.

**[Table 1]**

| | | Total RNA Quality check data | | | | | | Remarks | |
|---|---|---|---|---|---|---|---|---|---|
| | | Nano Drop (absorbance measurement) | | | | Bioanalyzer (electrophoresis) | | | |
| Sampl e No | Sample Name | Dilutio n ratio | Nucleic Acid conc. (ng/ul) | A260/ A280 | A2601 A230 | rRNA Ratio [28s/18s] | RNA Integrity Number (RIN) | Liquid volume (ul) | Amount of RNAs (ng) |
| 1 | Control | 1 | 188.9 | 1.90 | 1.24 | 0.00 | 2.6 | 15 | 2833.3 |
| 2 | Mo | 1 | 290.5 | 1.92 | 1.43 | 0.00 | 2.5 | 15 | 4357.6 |
| 3 | Apl | 1 | 179.7 | 1.89 | 1.03 | 0.00 | 2.6 | 15 | 2695.9 |
| 4 | D | 1 | 206.3 | 1.89 | 1.21 | 0.00 | 2.5 | 15 | 3094.4 |

### Example 4

### cDNA synthesis from nucleic acids extracted from samples

cDNA synthesis was conducted with the extracted RNAs using miScript II RT Kit (catalog No. 218160, Qiagen) under the following conditions.

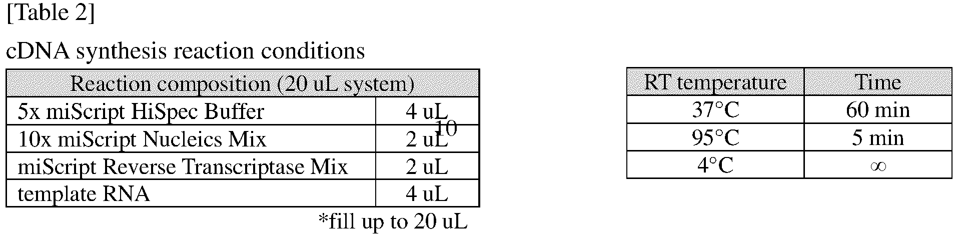

### Example 5

### Study on expression of miR-140 by real-time PCR

Real-time PCR reactions were conducted on a real-time PCR system (LightCycler 480, Roche) under the following conditions using miScript SYBR Green PCR Kit (catalog No. 218073, Qiagen) with the cDNAs synthesized as described above. The primer for the miR-140 measurement was Hs_miR-140_1 miScript Primer Assay (catalog No. MS00003500, has-miR-140-5p: MIMAT0000431).

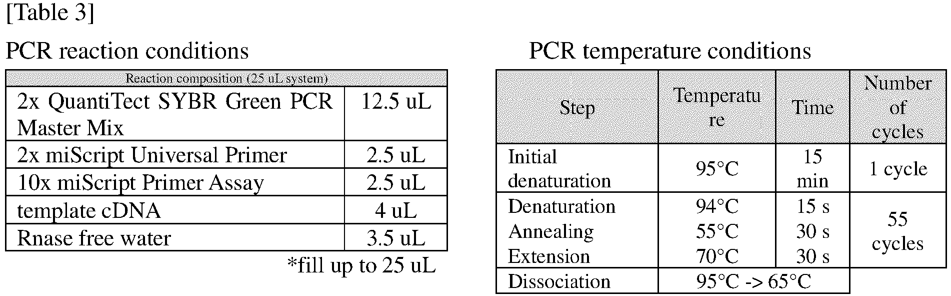

As a result, the following values were obtained. Consequently, the group treated with monensin was found to contain nearly twice as much miR-140 as the others. A low Cp value means that it was detected earlier and contains a large amount of miR-140.

**[Table 4]**

| | | Cp value | | | | Cp value Average | | | |
|---|---|---|---|---|---|---|---|---|---|
| Measurement site | Replicate | 1-Control | 2-Mo | 3-Apl | 4-D | 1-Control | 2-Mo | 3-Apl | 4-D |
| Hs miR-140_1 | n1 | 36.44 | 35.44 | 36.78 | 36.84 | 36.43 | 35.46 | 36.77 | 36.87 |
| | n2 | 36.42 | 35.47 | 36.76 | 36.89 | | | | |

### Discussion

Conventional anticancer drugs manifest their anticancer effects by inhibiting a single target molecule with one type of drug. Meanwhile, new in vivo substances that act on many different target molecular species with a single molecule have begun to be identified. Among them, micro-RNAs (miRs) have been found to inhibit the transcription or translation of various proteins, and an miR that has been shown to be effective in inhibiting cancer growth with experimental data is miR-140. This miR is released from cells in the form of exosomes, whose content is introduced into the surrounding cells without being decomposed even in the blood, and in those cells, the biosynthesis of proteins under their control is arrested. As a result, if these proteins are necessary for cancer growth, cancer cells that have introduced the content stop growing, leading to cancer treatment. Exosomes can also be effectively used for brain-related tumors as they can cross the blood-brain barrier.

Increasing miR-140 in various cancer cells has been reported to be effective in a wide range of cancers, including brain tumor, pharyngeal cancer, maxillary cancer, oral cancer, tongue cancer, lip cancer, thyroid cancer, lung cancer, breast cancer, esophageal cancer, stomach cancer, large intestine cancer, hepatocellular cancer, biliary tract cancer, pancreatic cancer, renal cell cancer, bladder cancer, cervical cancer, uterine corpus cancer, ovarian cancer, skin cancer, malignant melanoma, myeloid tumor, osteosarcoma, soft tissue tumor, angiosarcoma and pediatric solid tumor, etc., experimentally to date. Here, 42 types of cancer target molecules of effective miR-140 have been reported. The cancer target molecules include Muc1, Flap, CTSB, Yes, Wnt-1, Glut1, THY1, GRN, TGFBR, IGF1R, CeMP, Six1, Tripartivemotif28, ORC1, TRPM2, Brd9, cMet, CADM3, PD-L1, ZEB1, mTor, VEGF-A, MMP2, Pin1, HMGN, IL6, ADAMSTS5, IGFBP5, IGF2BP, IP3K, ADAM9, Sox4, Septin2, iASP, ADAM10, ATP8A, PDGFRA, MMO, FGF9, etc. PD-L1 and VEGF have already been used as antibody cancer drugs, although it is assumed that there is a significant difference in effectiveness between inhibiting molecular actions with an antibody and inhibiting the biosynthesis of such molecules. In addition, two expensive antibody drugs are also needed for these two target molecules. Antibody drugs also have a serious disadvantage of only being able to make antibodies against molecules that are either outside or on the surface of cells. In contrast, the miR/exosome drug can inhibit proteins such as enzymes, transcription factors and signaling factors that control various cancer target sites in a cell.

Simultaneously inhibiting these target molecules necessary for cancer growth, metastasis and survival clearly has a higher potential to improve anticancer effects compared to conventional molecular target drugs that are effective only in a single aspect, and exosomes containing miR-140 can also be extremely effective cancer drugs, as they can target a wide range of cancer types and cross the blood-brain barrier without being decomposed until they reach the target organ. Here, there are two possible methods of administration. One method is an intravenous injection, although in this case, the drug is distributed throughout the body but has a low rate of transfer into affected joints. In the case of cancer treatment, particularly when the cancer has already spread to the entire body, intravenous injections are appropriate, considering the possibility of metastasis to the brain. If the cancer has not yet spread, local administration to the affected site is preferred.

Meanwhile, the treatment of osteoarthritis, which tends to increase with age, is symptomatic, mainly using analgesics to relieve pain, with no drugs yet developed to cure the cause of the disease. However, a reduction in miR-140 has recently started to be reported in cases of osteoarthritis. Administrating exosomes containing miR-140 has also been reported to be effective in improving symptoms of osteoarthritis. For the administration for osteoarthritis treatment, intra-articular administration is considered to be preferred to increase the local concentration.

The challenge is where to stably obtain the exosomes containing miR-140. Firstly, articular cartilage has been reported to produce miR-140, and moreover, administrating the exosomes containing this miR to osteoarthritis patients has been reported to have a significant healing effect. Therefore, the inventors decided to collect exosomes containing miR-140 from human auricular chondrocytes, from which expression of miR-140 has not yet been reported. As miR-140 has been reported to be increased in production by estradiol (a female hormone), chondrocytes of female origin were used as starting cells. This resulted in obtaining cells having female hormone receptors, allowing the production of miR-140 to increase by adding estradiol to the culture system. Next, to stably collect exosomes from these cells, these cells were immortalized using Lenti-SV40 and Lenti-hTERT. Finally, to collect a large amount of exosomes from these cells, three exosome release enhancers were added before collecting exosomes, in which miR-140 was quantified. As a result, a previously unreported method to allow miR-140 to be abundantly and stably expressed in auricular chondrocytes has been determined. The exosomes containing miR-140 obtained in the above manner have the advantage of being effective as a drug as they can be stored for a long time in frozen form.

The invention can thus provide an exosome anticancer drug that is effective against various types of cancer with a single drug and the first exosome drug injectable into the joint cavity that offers regenerative properties to chondrocytes for osteoarthritis (OA).

### Industrial Applicability

The invention may be used in the pharmaceutical industry.

### Sequence Listing

20-223PCT.xml

## Claims

1. A pharmaceutical composition containing exosomes derived from auricular chondrocytes,
wherein the exosomes contain miR-140.

2. A method for producing a pharmaceutical composition having exosomes containing miR-140, comprising
an exosomes obtaining step of obtaining the exosomes from auricular chondrocytes.

3. The method for producing a pharmaceutical composition according to claim 2,
wherein the exosomes obtaining step comprises:
a culturing step of culturing auricular chondrocytes to obtain the cultured chondrocytes; and
an exosomes collecting step of collecting exosomes from the cultured chondrocytes cultured in the culturing step of the auricular chondrocytes,
wherein a culture medium for culturing auricular chondrocytes in the culturing step of the culturing auricular chondrocytes comprises estrogen.

4. The method for producing a pharmaceutical composition according to claim 3, wherein the estrogen comprises estradiol.

5. The method for producing a pharmaceutical composition according to claim 3,
wherein the exosomes collecting step comprises a step of collecting exosomes from the cultured chondrocytes using monensin or a monensin salt.

6. The method for producing a pharmaceutical composition according to claim 2,
wherein the exosomes obtaining step comprises:
a culturing step of auricular chondrocytes of culturing auricular chondrocytes to obtain cultured chondrocytes;
an immortalization step of immortalizing the cultured chondrocytes cultured in the culturing step of auricular chondrocytes to obtain immortalized cells; and
an exosomes collecting step of collecting exosomes from the immortalized cells.

7. The method for producing a pharmaceutical composition according to claim 6,
wherein the immortalization step comprises a step of immortalizing the cultured chondrocytes using SV40 or hTERT to obtain immortalized cells.

8. The method for producing a pharmaceutical composition according to claim 6,
wherein the exosomes collecting step comprises a step of collecting exosomes from the immortalized cells using monensin or a monensin salt.
